# EUROPÄISCHE PATENTSCHRIFT

(11) **EP 3 558 921 B1**
(45) Veröffentlichungstag und Bekanntmachung des Hinweises auf die Patenterteilung: **14.10.2020**
(21) Anmeldenummer: 17811596.0
(22) Anmeldetag: 11.12.2017
(51) Int. Cl.: C07C 67/54, C07C 69/54

(54) **VERFAHREN ZUR DESTILLATIVEN GEWINNUNG VON REIN-BUTYLACRYLAT AUS ROH-BUTYLACRYLAT, WOBEI BUTYL FÜR N-BUTYL ODER ISO-BUTYL STEHT**
METHOD FOR THE RECOVERY OF PURE BUTYL ACRYLATE FROM RAW BUTYL ACRYLATE BY MEANS OF DISTILLATION, WHEREIN BUTYL STANDS FOR N-BUTYL OR ISOBUTYL
PROCÉDÉ DE PRODUCTION PAR DISTILLATION DE BUTYLACRYLATE PUR À PARTIR DE BUTYLACRYLATE BRUT, LE BUTYLE ÉTANT DU N-BUTYLE OU DE L'ISOBUTYLE

(30) Priorität: 21.12.2016 EP 16205953
(43) Veröffentlichungstag der Anmeldung: 30.10.2019
(73) Patentinhaber: BASF SE, 67056 Ludwigshafen am Rhein (DE)
(72) Erfinder: LANG, Ortmund, 67056 Ludwigshafen (DE); METZEN, Bernd, 67056 Ludwigshafen (DE); HECHLER, Claus, 67056 Ludwigshafen (DE)
(74) Vertreter: BASF IP Association
(86) Internationale Anmeldenummer: PCT/EP2017/082186
(87) Internationale Veröffentlichungsnummer: WO 2018/114422

(56) Entgegenhaltungen:
- JP-A- 2005 239 564

## Beschreibung

Ester der Acrylsäure, H₂C=CH-C(=O)OR, werden im Folgenden auch Acrylate genannt. R steht für einen Alkylrest.

Acrylate wie zum Beispiel n-Butylacrylat werden großtechnisch in der Regel durch Umsetzung von Alkohol (Beispiel: n-Butanol) und Acrylsäure gewonnen. Bei der Synthese (Veresterungsreaktion) entsteht ein Produktgemisch, auch Roh-Acrylat genannt, worin das Acrylat in der Regel überwiegt.

Von Interesse sind n-Butyl- und iso-Butylacrylat. Damit entspricht R n-Butyl bzw. iso-Butyl.

Butylacrylate finden Anwendung für Lacke, Klebstoffe, Bauchemikalien, Papierbeschichtungen und Kunststoffe.

Um den Spezifikationsanforderungen zu entsprechen, muss das in einer Synthese anfallende Roh-Acrylat weiter destillativ gereinigt werden. Die Spezifikationsanforderungen für reine Acrylate sehen z.B. insbesondere einen Mindestgehalt an Acrylat von 99,5 Gew.-% und einen maximal zulässigen Gehalt an Acetat, RO(C=O)CH₃, von 1500 ppm und an Diether, ROR, von 1000 ppm vor.

Die Gewinnung von Acrylat aus dem Roh-Acrylat ist wegen der geringen Unterschiede in den relativen Flüchtigkeiten der Komponenten ein kompliziertes destillationstechnisches Problem und wird daher in der Regel durch eine Zweikolonnenschaltung durchgeführt (s.u.). Aufgrund der Empfindlichkeit der zur Polymerisation neigenden Acrylate sind besondere Kolonneneinbauten in der Regel besonders vorteilhaft.

Die destillative Gewinnung von Acrylat aus Roh-Acrylat erfolgt nach einer bevorzugt durchgeführten Vorreinigung, z.B. durch Extraktion, im Stand der Technik, wie er z.B. in der Dissertation 'Polymerisationsinhibierung von (Meth-)Acrylaten', TU Darmstadt (Fachbereich Chemie), 2003, von Holger Becker auf den Seiten 21 bis 24 dargestellt wird, in zwei hintereinandergeschalteten Destillationskolonnen:
In einer ersten Destillationskolonne wird ein Gemisch von überwiegend Niedersiedern (bez. auf das Butylacrylat), wie z.B. Wasser, Alkohol (ROH), Acetat (RO(C=O)CH₃), Diether (ROR), als Kopfprodukt abgezogen, wobei die organischen Niedersieder zur Veresterung zurückgeführt werden können. Im Sumpf wird das Acrylat und die Höhersieder abgetrennt. In einer zweiten nachgeschalteten Destillationskolonne werden als Sumpfprodukt die Höhersieder (bez. auf das Butylacrylat), wie z.B. Butoxyester (ROCH₂CH₂C(=O)OR), abgetrennt. Das Wertprodukt (also das Rein-Butylacrylat) wird als Kopfprodukt der zweiten Destillationskolonne entnommen. Vgl. die Kolonnen H und I in Abb. 3-7 auf Seite 24 der o.g. Dissertation. Der Sumpfablauf der Kolonne I wird einem Schwersiederabscheider J zugeführt (vgl. Abb. 3-7 auf Seite 24 der o.g. Dissertation).

DE 3302525 A1 (BASF AG) sowie die Fachliteratur, beispielsweise Kaibel et al. in Chem. Eng. Technol. 10 (1987), Seiten 92 bis 98, und in Chem. Ing.-Tech. 61 (1989), Nr. 2, Seiten 104 bis 112, beschreiben allgemein die Anwendung von Trennwandkolonnen bei der destillativen Reinigung von organischen Verbindungen.

US 2013/0284586 A1 (LG Chem. Ltd.) beschreibt die Verwendung einer Trennwandkolonne zur destillativen Reinigung von 2-Ethylhexyl-acrylat.

JP 2005/239564 A (Mitsubishi Rayon Co.) beschreibt die Destillation von (Meth)acrylsäureestern unter Verwendung einer Trennwandkolonne.

Demgegenüber lag der Erfindung die Aufgabe zugrunde, ein verbessertes Verfahren zur destillativen Gewinnung von Rein-Butylacrylat, nämlich n-Butyl- und iso-Butylacrylat, aus dem entsprechenden Roh-Butylacrylat zur Verfügung zu stellen, das, bei Einhaltung der jeweiligen geforderten Spezifikationen für das Rein-Butylacrylat, wirtschaftlicher ist, insbesondere bezüglich der Investitions- und Energiekosten.

Demgemäß wurde ein Verfahren zur destillativen Gewinnung von Rein-Butylacrylat aus Roh-Butylacrylat gefunden, wobei Butyl für n-Butyl oder iso-Butyl steht, welches dadurch gekennzeichnet ist, dass das Verfahren in einer Trennwandkolonne (1) mit trennwirksamen Einbauten und Verdampfer (7) durchgeführt wird, in der eine Trennwand (8) in Kolonnenlängsrichtung unter Ausbildung eines oberen gemeinsamen Kolonnenbereichs (9), eines unteren gemeinsamen Kolonnenbereichs (14), eines Zulaufteils (10, 12) mit einer Seitenzulaufstelle (2) und eines Entnahmeteils (11, 13) mit einer Seitenentnahmestelle (3) angeordnet ist, die Kolonne eine Anzahl von theoretischen Trennstufen im Bereich von 20 bis 80 aufweist, die Seitenzulaufstelle (2) für das Roh-Butylacrylat auf einer theoretischen Trennstufe im Bereich beginnend mindestens zwei theoretische Trennstufen oberhalb der untersten und endend mindestens zwei theoretische Trennstufen unterhalb der obersten theoretischen Trennstufe angeordnet ist, die Seitenentnahmestelle (3) für das Rein-Butylacrylat auf einer theoretischen Trennstufe im Bereich beginnend mindestens zwei theoretische Trennstufen oberhalb der untersten und endend mindestens zwei theoretische Trennstufen unterhalb der obersten theoretischen Trennstufe angeordnet ist und die Trennwand (8) in der Kolonne im Bereich beginnend mindestens eine theoretische Trennstufe oberhalb der untersten und endend mindestens eine theoretische Trennstufe unterhalb der obersten theoretischen Trennstufe angeordnet ist, wobei man das Flüssigkeitsmengenverhältnis am oberen Ende der Trennwand (8) auf den Verstärkungsteil (10) und den Abtriebsteil (11) der Kolonne in einem Bereich von 1 : 0,2 bis 1 : 5 und das Mengenverhältnis der Brüdenströme am unteren Ende der Trennwand (8) auf den Abtriebsteil (12) und den Verstärkungsteil (13) der Kolonne in einem Bereich von 1 : 0,5 bis 1 : 2,0 einstellt, und wobei es sich bei dem Butylacrylat um n-Butylacrylat oder iso-Butylacrylat handelt.

Unter Rein-Butylacrylat ist besonders ein Butylacrylat mit einer Reinheit von ≥ 98,5 Gew.-%, insbesondere von ≥ 99,5 Gew.-%, n-Butylacrylat bzw. iso-Butylacrylat zu verstehen.

Das im erfindungsgemäßen Verfahren eingesetzte Roh-n-Butylacrylat weist die folgende Zusammensetzung auf:
40 bis 90 Gew.-%, insbesondere 60 bis 90 Gew.-%, n-Butylacrylat,
0,1 bis 20 Gew.-%, insbesondere 1 bis 10 Gew.-%, n-Butanol,
0,1 bis 20 Gew.-%, insbesondere 0,5 bis 10 Gew.-%, Wasser,
0,1 bis 20 Gew.-%, insbesondere 1 bis 10 Gew.-%, Höhersieder (bez. auf n-Butylacrylat),
0,1 bis 20 Gew.-%, insbesondere 1 bis 10 Gew.-%, weitere Niedersieder (bez. auf n-Butylacrylat).

Das im erfindungsgemäßen Verfahren eingesetzte Roh-iso-Butylacrylat weist die folgende Zusammensetzung auf:
40 bis 90 Gew.-%, insbesondere 60 bis 90 Gew.-%, iso-Butylacrylat,
0,1 bis 20 Gew.-%, insbesondere 1 bis 10 Gew.-%, iso-Butanol,
0,1 bis 20 Gew.-%, insbesondere 0,5 bis 10 Gew.-%, Wasser,
0,1 bis 20 Gew.-%, insbesondere 1 bis 10 Gew.-%, Höhersieder (bez. auf iso-Butylacrylat),
0,1 bis 20 Gew.-%, insbesondere 1 bis 10 Gew.-%, weitere Niedersieder (bez. auf iso-Butylacrylat).

Das erfindungsgemäße Verfahren wird in einer Trennwandkolonne (1) durchgeführt, in der eine Trennwand (8) in Kolonnenlängsrichtung unter Ausbildung eines oberen gemeinsamen Kolonnenbereichs (9), eines unteren gemeinsamen Kolonnenbereichs (14), eines Zulaufteils (10, 12) und eines Entnahmeteils (11, 13) angeordnet ist.

Es wurde überraschend gefunden, dass die destillative Gewinnung von Rein-Butylacrylat aus Roh-Butylacrylat entgegen der Annahme, dass eine zweistufige Betriebsweise mit unterschiedlichen Drücken erforderlich sei, in einer einzigen Kolonne, und zwar einer Trennwandkolonne und somit bei einheitlichem Druck durchgeführt werden kann.

Eine Trennwandkolonne ist eine Destillationskolonne mit senkrechter Trennwand, die in Teilbereichen eine Quervermischung von Flüssigkeits- und Brüdenströmen verhindert. Die Trennwand, die i.d.R. aus einem ebenen Blech besteht und geschweißt, geschraubt oder gesteckt sein kann, unterteilt die Kolonne in Längsrichtung in deren mittlerem Bereich in einen Zulaufteil und einen Entnahmeteil. Das aufzutrennende Gemisch, das Roh-Butylacrylat, wird dem Zulaufteil zugeführt und das Produkt, das Rein-Butylacrylat, wird aus dem Entnahmeteil abgezogen.

Das Verfahren wird bevorzugt kontinuierlich durchgeführt.

Die Trennwandkolonne ist, wie in der Regel jede Destillationskolonne, mit einem Verdampfer (Sumpfverdampfer) (7) und einem Kondensator (6) am Kolonnenkopf ausgestattet.

Im erfindungsgemäßen Verfahren wird in vorteilhafter Weise die Verweilzeit im Verdampfer (7) und dem zugehörigen Rohrleitungssystem bevorzugt auf 1 bis 60 Minuten, weiter bevorzugt auf 10 bis 30 Minuten, begrenzt. Dadurch wird trotz der Polymerisationsanfälligkeit des Gemisches ein störungsfreier Betrieb der Anlage, insbesondere nur geringfügige oder keine Verschmutzungen, gewährleistet.

Erfindungsgemäß wird das Flüssigkeitsmengenverhältnis am oberen Ende der Trennwand (8) auf den Verstärkungsteil (10) und den Abtriebsteil (11) der Kolonne, also Menge auf Verstärkungsteil (10) : Menge auf Abtriebsteil (11), in einem Bereich von 1 : 0,2 bis 1 : 5, also 5 bis 0,2, bevorzugt in einem Bereich von 1 : 0,5 bis 1 : 2, also 2 bis 0,5, eingestellt. Dies erfolgt bevorzugt in der Weise, dass die Flüssigkeit am oberen Ende der Trennwand gesammelt und über eine Regelungs- oder Stellvorrichtung im genannten Verhältnis auf den Verstärkungs- bzw. Abtriebsteil der Kolonne aufgegeben wird. Dadurch wird ein niedrigerer Energieverbrauch gewährleistet.

Erfindungsgemäß wird zusätzlich zur Regelung des Flüssigkeitsmengenrücklaufverhältnisses am oberen Ende der Trennwand (8) auch das Mengenverhältnis der Brüdenströme (= Dampfströme) am unteren Ende der Trennwand (8) auf den Abtriebsteil (12) und auf den Verstärkungsteil (13) der Kolonne eingestellt. Dies erfolgt bevorzugt durch Wahl der Trenneinbauten und/oder durch den zusätzlichen Einbau druckverlusterzeugender Einbauten, beispielsweise von Blenden oder durch eine Mengenregelung der Brüdenströme.

Erfindungsgemäß werden die Mengen der Brüdenströme auf den Abtriebsteil (12) und den Verstärkungsteil (13) der Kolonne, also Menge auf Abtriebsteil (12) : Menge auf Verstärkungsteil (13), in einem Verhältnis im Bereich von 1 : 0,5 bis 1 : 2,0, also 2 bis 0,5, bevorzugt in einem Verhältnis im Bereich von 1 : 0,9 bis 1 : 1,5, also 1/0,9 bis 1/1,5, eingestellt.

Das erfindungsgemäße Verfahren wird bevorzugt bei einem Druck im Kolonnenkopf von 20 mbar bis 5 bar, bevorzugt von 50 bis 200 mbar, durchgeführt.

Bevorzugt ist im oberen gemeinsamen Kolonnenbereich (9) eine Temperaturregelung vorgesehen, mit einem Temperatursignal, welches von einer einzelnen oder gemittelt von mehreren Messstellen unterhalb der obersten theoretischen Trennstufe, bevorzugt auf der dritten theoretischen Trennstufe von oben gezählt, stammen kann, die als Stellgröße den Destillatstrom, das Rücklaufverhältnis oder bevorzugt die Rücklaufmenge, nutzt.

Dadurch wird ein stabiler Betrieb der Kolonne gewährleistet mit der Folge einer weiteren Verbesserung der erzielbaren Produktreinheit.

In einer weiteren Verfahrensvariante ist zusätzlich oder alternativ eine Temperaturregelung im unteren Kolonnenbereich vorgesehen, mit einem Temperatursignal, welches von einer einzelnen oder gemittelt von mehreren Messstellen oberhalb der untersten theoretischen Trennstufe, bevorzugt auf der zweiten theoretischen Trennstufe von unten gezählt, stammen kann, die als Stellgröße die Sumpfentnahmemenge nutzt. Durch diese zusätzliche Maßnahme wird eine weitere Verbesserung des stabilen Kolonnenbetriebs erreicht. Darüber hinaus ist es zusätzlich oder alternativ möglich, eine Standregelung am Kolonnensumpf vorzusehen, die als Stellgröße die Seitenentnahmemenge nutzt.

Das Verhältnis der Querschnittsflächen des Bereichs des Entnahmeteils (11, 13) zum Bereich des Zulaufteils (10, 12) liegt bevorzugt bei 4 : 1 bis 1 : 4, besonders bevorzugt bei 1,5 : 1 bis 1 : 1,5, z.B. bei 1 : 1.

Die Trennwandkolonne (1) weist eine Anzahl von theoretischen Trennstufen im Bereich von 20 bis 80 auf.

Trennwirksame Einbauten befinden sich im gemeinsamen oberen Kolonnenbereich (9) und im gemeinsamen unteren Kolonnenbereich (14) sowie im Zulaufteil (10, 12) und Entnahmeteil (11, 13).

Die Angabe der Anzahl der theoretischen Trennstufen der Trennwandkolonne (1) bezieht sich stets auf die Summe der theoretischen Trennstufen im gemeinsamen oberen Kolonnenbereich (9), dem gemeinsamen unteren Kolonnenbereich (14) und dem Zulaufteil (10, 12).

In der Regel ist die Anzahl der theoretischen Trennstufen im Entnahmeteil (11, 13) gleich groß wie im Zulaufteil (10, 12), kann jedoch auch größer, z.B. um 1 bis 5 größer, oder kleiner, z.B. um 1 bis 5 kleiner, sein.

Die Seitenzulaufstelle (2) für das Roh-Butylacrylat ist auf einer theoretischen Trennstufe im Bereich beginnend mindestens zwei theoretische Trennstufen oberhalb der untersten und endend mindestens zwei theoretische Trennstufen unterhalb der obersten theoretischen Trennstufe, bevorzugt auf einer theoretischen Trennstufe im Bereich beginnend mindestens fünf theoretische Trennstufen oberhalb der untersten und endend mindestens fünf theoretische Trennstufen unterhalb der obersten theoretischen Trennstufe, angeordnet.

Die Seitenentnahmestelle (3) für das Rein-Butylacrylat ist auf einer theoretischen Trennstufe im Bereich beginnend mindestens zwei theoretische Trennstufen oberhalb der untersten und endend mindestens zwei theoretische Trennstufen unterhalb der obersten theoretischen Trennstufe, bevorzugt im Bereich beginnend mindestens fünf theoretische Trennstufen oberhalb der untersten und endend mindestens fünf theoretische Trennstufen unterhalb der obersten theoretischen Trennstufe, angeordnet.

Die Trennwand (8) ist in der Kolonne im Bereich beginnend mindestens eine theoretische Trennstufe oberhalb der untersten und endend mindestens eine theoretische Trennstufe unterhalb der obersten theoretischen Trennstufe, bevorzugt im Bereich beginnend mindestens vier theoretische Trennstufen oberhalb des untersten und endend mindestens vier theoretische Trennstufen unterhalb der obersten theoretischen Trennstufe, besonders bevorzugt jeweils mittig, angeordnet.

In einer besonders bevorzugten Ausführungsform weist die Trennwandkolonne (1) eine Anzahl von theoretischen Trennstufen im Bereich von 30 bis 40 auf, die Seitenzulaufstelle (2) für das Roh-Butylacrylat ist auf einer theoretischen Trennstufe im Bereich beginnend mindestens 20 theoretischen Trennstufen oberhalb der untersten und endend mindestens zwei theoretische Trennstufen unterhalb der obersten theoretischen Trennstufe angeordnet, die Seitenentnahmestelle (3) für das Rein-Butylacrylat ist auf einer theoretischen Trennstufe im Bereich beginnend mindestens 10 theoretische Trennstufen oberhalb der untersten und endend mindestens 10 theoretische Trennstufen unterhalb der obersten theoretischen Trennstufe angeordnet und die Trennwand (8) in der Kolonne ist im Bereich beginnend mindestens fünf theoretische Trennstufen oberhalb des untersten und endend mindestens fünf theoretische Trennstufen unterhalb der obersten theoretischen Trennstufe angeordnet.

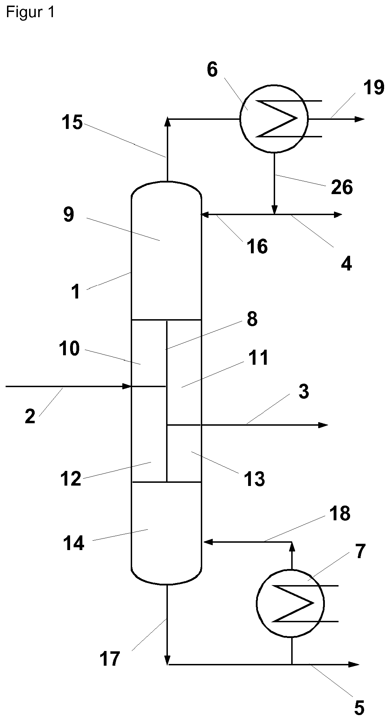

Im Fall von gleicher Anzahl an theoretischen Trennstufen im Entnahmeteil (11, 13) wie im Zulaufteil (10, 12) kann die Seitenentnahmestelle (3) sowohl auf der gleichen theoretischen Trennstufe wie die Seitenzulaufstelle (2) als auch unter- oder oberhalb der Seitenzulaufstelle liegen; aber selbstverständlich jeweils auf der anderen Seite der Trennwand (8) (vgl. Figur 1); bevorzugt liegt die gegenüberliegende Seitenentnahmestelle (3) unterhalb, z.B. eine bis 25, besonders 5 bis 20, ganz besonders 10 bis 18, theoretische Trennstufen unterhalb, der Seitenzulaufstelle (2). (Das Zählen der Trennstufen erfolgt in der Kolonne bzw. im betreffenden Kolonnenbereich bzw. im betreffenden Kolonnenteil immer von unten nach oben).

Im Fall verschiedener Anzahl an theoretischen Trennstufen im Entnahmeteil (11, 13) wie im Zulaufteil (10, 12) wird zur Zählung der Anzahl theoretischer Trennstufen für die Festlegung der relativen Höhenposition von Zulauf- und Entnahmestelle diejenige Seite mit der höheren Gesamtanzahl an Trennstufen im Bereich der Trennwand (8) herangezogen.

Bezüglich der trennwirksamen Einbauten gibt es grundsätzlich keine Einschränkungen; bevorzugt sind Füllkörper und/oder geordnete Packungen und/oder Böden vorgesehen.

Die Einbauten können jeweils individuell für die Kolonnenbereiche oberhalb und unterhalb (9, 14) sowie in den Bereichen der Trennwand (10, 11, 12, 13) gewählt werden.

In einer weiteren bevorzugten Verfahrensvariante werden Dual-Flow-Böden als trennwirksame Einbauten in der Trennwandkolonne eingesetzt. Der Begriff Dual-Flow-Boden bezeichnet in bekannter Weise einen Kolonnenboden mit Öffnungen, die von Dampf und Flüssigkeit im Gegenstrom passiert werden.

Bei der thermischen Behandlung von Gemischen, die eine oder mehrere polymerisierbare Verbindungen enthalten, in einer Kolonne, besteht immer das Problem, dass die Kolonne und die Kolonneneinbauten durch Ablagerungen verschmutzen und aufwendig gereinigt werden müssen, mit der Folge, dass der Betrieb unterbrochen werden muss. Als thermische Behandlung werden vorliegend Verfahren wie Destillation oder Rektifikation, Absorption, Extraktion oder Strippen verstanden. Gemische, die der thermischen Behandlung in einer Kolonne unterworfen werden können, sind in der Regel fluid, d.h. gasförmig, flüssig oder gasförmig/flüssig.

Durch den Einsatz von Dual-Flow-Böden wird Verschmutzungsanfälligkeit der Trennwandkolonne gegenüber herkömmlichen Bodenkolonnen vermindert. Dadurch verlängert sich die Betriebszeit der Kolonne und ihre Wirtschaftlichkeit ist somit erhöht.

Bevorzugt werden Dual-Flow-Böden im Bereich der Trennwand (10, 11, 12, 13) eingesetzt; in einer weiter bevorzugten Ausführungsform werden Dual-Flow-Böden auch im gemeinsamen oberen Kolonnenbereich (9) und im gemeinsamen unteren Kolonnenbereich (14) eingesetzt. Eine weitere vorteilhafte Ausführungsform ist der Einsatz von Dual-Flow-Böden im Bereich der Trennwand (10, 11, 12, 13) und im gemeinsamen unteren Kolonnenbereich (14) sowie der Einsatz von Füllkörpern oder Packungen im gemeinsamen oberen Kolonnenbereich (9).

In der WO 03/043712 A1 (BASF AG) wurde für eine konventionelle Kolonne ohne Trennwand gezeigt, dass durch gezielte Ausgestaltung der Durchmesser der Öffnungen in den Dual-Flow-Böden eine erhebliche Reduzierung der Verschmutzungsanfälligkeit und somit eine erhebliche Verlängerung der Betriebszeit von Bodenkolonnen erreicht werden konnte.

Für Trennwandkolonnen gilt, dass über beide Seiten der Trennwand der gleiche Druckverlust herrscht. Eine genaue Einstellung der Gasaufteilung über die jeweiligen Böden auf der Zulaufseite und auf der Entnahmeseite ist durch die Wahl der Öffnungsverhältnisse der Böden auf der Zulaufseite und auf der Entnahmeseite von großem Vorteil.

Durch die gezielte Ausgestaltung der Öffnungsverhältnisse kann die Gasaufteilung zu der Zulaufseite und der Entnahmeseite genau eingestellt werden. Durch die unterschiedlichen Öffnungsverhältnisse der Dual-Flow-Böden entstehen bei gleichem Druckverlust unterschiedliche Gasmengen auf beiden Seiten der Trennwand. Dadurch kann auf eine aufwendige Gasverteilung unterhalb der Trennwand verzichtet werden.

Das Öffnungsverhältnis wird über die Größe und/oder Anzahl der Öffnungen eingestellt. Als Öffnungsverhältnis eines Dual-Flow-Bodens wird in bekannter Weise das Verhältnis aus der Summe der Öffnungsflächen und der Gesamtfläche des Dual-Flow-Bodens bezeichnet.

Erfindungsgemäß können die Öffnungen der Dual-Flow-Böden innerhalb einer Kolonne unterschiedlich ausgestaltet, und zwar dergestalt, dass der Durchmesser der Öffnungen und/oder die Anzahl der Öffnungen variiert werden.

Es besteht grundsätzlich keine Einschränkung bezüglich der Form der Öffnungen:
Diese können jede geometrische Form, beispielsweise Kreise, Ellipsen, Rechtecke oder Polygone, aufweisen. Bevorzugt sind die Öffnungen in den Dual-Flow-Böden kreisförmig.

Der Fachmann kann in Abhängigkeit von Gas- und Flüssigkeitsbelastung sowie Öffnungsdurchmesser das erforderliche Öffnungsverhältnis leicht bestimmen. Der Durchmesser der Öffnungen in den Dual-Flow-Böden liegt bevorzugt im Bereich von 10 bis 80 mm, wobei oberhalb des Zulaufs angeordnete Dual-Flow-Böden bevorzugt Öffnungen im Bereich von 10 bis 50 mm, unterhalb des Zulaufs angeordnete Dual-Flow-Böden dagegen bevorzugt Öffnungen mit Durchmessern im Bereich von 15 bis 80 mm aufweisen.

Das Öffnungsverhältnis der Dual-Flow-Böden liegt bevorzugt im Bereich von 10 bis 30 %.

Im erfindungsgemäßen Verfahren wird das Acrylmomonere, d.h. das Butylacrylat, bevorzugt durch geeignete Polymerisationsinhibitoren stabilisiert, um eine ungewünschte Polymerisation zu vermeiden. D.h., das erfindungsgemäße Verfahren wird bevorzugt in Gegenwart wirksamer Mengen eines Stabilisators oder mehrerer Stabilisatoren durchgeführt. Als Stabilisatoren eignen sich prinzipiell alle Polymerisationsinhibitoren, die zur Stabilisierung von (Meth)acrylsäure und (Meth)acrylsäureestern in z.B. DE 10 2005 053 982 A1 (BASF AG) und DE 102 58 329 A1 (BASF AG) empfohlen werden.

Geeignete Stabilisatoren können beispielsweise N-Oxide (Nitroxyl- oder N-Oxyl-Radikale, also Verbindungen, die wenigstens eine >N-O-Gruppe aufweisen), wie z.B. 4-Hydroxy-2,2,6,6-tetramethylpiperidin-N-oxyl oder 4-Oxo-2,2,6,6-tetramethylpiperidin-N-oxyl, Phenole und Naphthole, wie p-Methoxyphenol, p-Aminophenol, p-Nitrosophenol, 2-tert.-Butylphenol, 4-tert.-Butylphenol, 2,4-Di-tert.-butylphenol, 2-Methyl-4-tert.-butylphenol, 2,6-tert.-Butyl-4-methylphenol oder 4-tert.-Butyl-2,6-dimethylphenol, Chinone, wie z.B. Hydrochinon oder Hydrochinonmonomethylether, aromatische Amine, wie z.B. N,N-Diphenylamin, Phenylendiamine, wie z.B. N,N'-Dialkyl-p-phenylendiamin, wobei die Alkylreste gleich oder verschieden sein können und jeweils unabhängig voneinander 1 bis 4 Kohlenstoffatome aufweisen und geradkettig oder verzweigt sein können, wie z.B. N,N'-Dimethyl-p-phenylendiamin oder N,N'-Diethyl-p-phenylendiamin, Hydroxylamine, wie z.B. N,N-Diethylhydroxylamin, Imine, wie z.B. Methylethylimin oder Methylenviolett, Sulfonamide, wie z.B. N-Methyl-4-toluolsulfonamid oder N-tert.-Butyl-4-toluolsulfonamid, Oxime, wie Aldoxime, Ketoxime oder Amidoxime, wie z.B. Diethylketoxim, Methylethylketoxim oder Salicylaldoxim, phosphorhaltige Verbindungen, wie z.B. Triphenylphosphin, Triphenylphosphit oder Triethylphosphit, schwefelhaltige Verbindungen wie z.B. Diphenylsulfid oder Phenothiazin, Metallsalze, wie z.B. Cer(III)acetat oder Cer(III)ethylhexanoat, oder Gemische davon, sein. Bevorzugt erfolgt die Stabilisierung mit Phenothiazin (PTZ), p-Methoxyphenol (MeHQ), Hydrochinon, Hydrochinonmonomethylether, 4-Hydroxy-2,2,6,6-tetramethylpiperidin-N-oxyl, 4-Oxo-2,2,6,6-tetramethylpiperidin-N-oxyl, 2,6-tert.-Butyl-4-methylphenol oder Gemischen davon. Ganz besonders bevorzugt wird Phenothiazin (PTZ) und/oder p-Methoxyphenol (MeHQ) als Polymerisationsinhibitor verwendet.

Auch wenn die Inhibitoren als Reinsubstanz zugegeben werden können, ist es von Vorteil, den Inhibitor gelöst in einem Lösungsmittel als einfach und reproduzierbar zu dosierende Lösung zuzugeben, wobei grundsätzlich auch Inhibitormischungen in einer einzelnen Lösung möglich sind. Bevorzugt wird eine im Acrylatsyntheseverfahren bzw. dem Stoffgemisch in der Kolonne bereits vorhandene Flüssigkeit als Lösungsmittel verwendet. Besonders bevorzugt für die Wahl als Lösungsmittel ist das Acrylatprodukt selbst (hier n-Butyl- bzw. iso-Butylacrylat) oder einer der Syntheseeinsatzstoffe für das Acrylat (hier Acrylsäure oder n-Butanol bzw. iso-Butanol).

Die Erfindung wird im Folgenden anhand einer Zeichnung (Figur 1) und eines Ausführungsbeispiels näher erläutert.

Die Zeichnung zeigt in der einzigen Figur eine Trennwandkolonne 1 mit Trennwand 8, die die Trennwandkolonne 1 in einen gemeinsamen oberen Kolonnenbereich 9, einen Zulaufteil 10 und 12, mit Verstärkungsteil 10 und Abtriebsteil 12, einen Entnahmeteil 11 und 13 mit einem Abtriebsteil 11 und einem Verstärkungsteil 13, sowie einen gemeinsamen unteren Kolonnenbereich 14 unterteilt. Trennwirksame Einbauten befinden sich in den Kolonnenbereichen 9 und 14 und in den Teilen 10 bis 13. Das Roh-Butylacrylat 2 tritt zwischen den Kolonnenteilen 10 und 12 in die Trennwandkolonne 1 ein. Das Rein-Butylacrylat 3 wird zwischen den Kolonnenteilen 11 und 13, bevorzugt in flüssiger Form, entnommen. Der am Kolonnenkopf anfallende Brüdenstrom 15 wird im Kondensator 6, der gegebenenfalls durch einen Nachkühler ergänzt wird, teilweise kondensiert und in den Rücklaufstrom 16 sowie den Destillatstrom 4 aufgeteilt. Der nicht kondensierte Anteil aus dem Kondensator 6 enthält die niedersiedenden Verunreinigungen und wird dampfförmig als Strom 19 abgezogen. Am unteren Kolonnenende wird die Flüssigkeit 17 in einem Verdampfer 7 teilweise verdampft und über die Rohrleitung 18 in die Kolonne zurückgeführt. Ein Teilstrom 5, der die höhersiedenden Verunreinigungen enthält, wird abgezogen. Der Verdampfer 7 kann als Naturumlaufverdampfer oder als Zwangsumlaufverdampfer ausgebildet sein, im letzteren Fall ist zusätzlich eine Umlaufpumpe für den Flüssigkeitsstrom 17 erforderlich. Besonders vorteilhaft hinsichtlich der Vermeidung unerwünschter Polymerisationsreaktionen ist es anstelle des Zwangsumlaufverdampfers einen Fallfilmverdampfer einzusetzen, da mit dieser Bauart die kürzesten Verweilzeiten möglich sind. Zur Verringerung der Verweilzeit der Flüssigkeit im Verdampfersystem ist es günstig, die Standhaltung nicht in der unteren Kolonnenhaube, sondern in der Zulaufleitung der Flüssigkeit 17 anzuordnen.

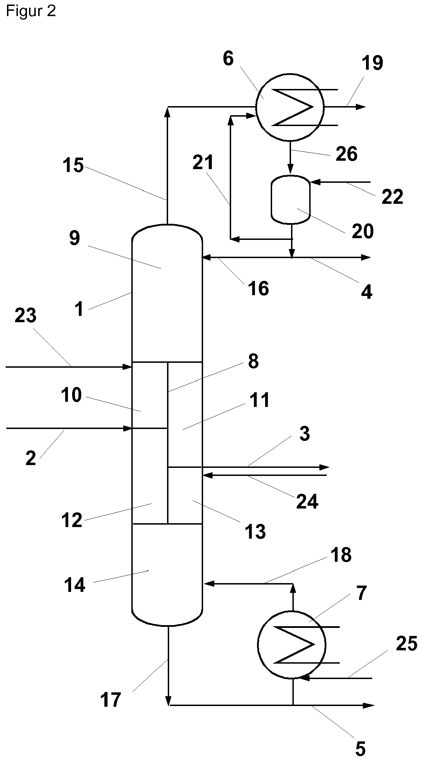

In einer bevorzugten Fahrweise (vgl. Figur 2) wird im erfindungsgemäßen Verfahren ein Stabilisator 1 (23) (sog. Prozessstabilisator; z.B. insbesondere PTZ) in den Verstärkungsteil (10) des Zulaufteils (10, 12), dort im Besonderen knapp unterhalb des oberen Endes der Trennwand (8), zugegeben. Der Stabilisator 1 kann insbesondere als Lösung in einem geeigneten Lösungsmittel, besonders wie oben aufgeführt, z.B. n-Butylacrylat bzw. iso-Butylacrylat, eingesetzt werden. Dadurch ist der gesamte Zulaufteil (10, 12) und der gemeinsame untere Teil der Kolonne (14) mit dem Prozessstabilisator stabilisiert. (Unter 'knapp unterhalb des oberen Endes der Trennwand (8)' ist z.B. ,eine bis 5 theoretische Trennstufen unterhalb des oberen Endes der Trennwand (8)' zu verstehen).

Weiterhin (vgl. Figur 2) wird im erfindungsgemäßen Verfahren bevorzugt ein Stabilisator 2 (22) (sog. Lagerstabilisator; z.B. insbesondere MeHQ) in den Behälter (20), der das Kondensat (26) auffängt, und/oder in die Leitung eines Quenchkreislaufs (21) und/oder am Kopf des Kondensators (6) zugegeben. Der bevorzugt eingerichtete Quenchkreislauf (d.h der flüssige Rückführstrom eines Teils des Kondensats, z.B. 10 bis 50 Gewichtshundertstel des Kondensats, in den Kondensator (6)) hat die Funktion, dass die natürlicherweise stabilisatorfreien Brüden (15) beim Kondensieren im Kondensator (6) besonders ausreichend stabilisiert sind. Über die Rücklaufleitung (16) wird dann der gemeinsame obere Kolonnenbereich (9) oberhalb der Trennwand (8) sowie Zulaufteil (10, 12) und Entnahmeteil (11, 13) im Bereich der Trennwand mit dem Stabilisator (insb. MeHQ) stabilisiert, wobei auch Sauerstoff zugegen ist, der aus Magerluft stammt. Die Zufuhr von Magerluft (25) (Mischung aus Luft und Stickstoff, insbesondere derart, dass ein Sauerstoffgehalt von 4 bis 9 Vol.% resultiert) erfolgt besonders entweder am unteren Ende des Verdampfers (7) oder am unteren Ende der Kolonne (1).

In einer weiteren Verfahrensvariante (vgl. Figur 2) wird in den Verstärkungsteil (13) unterhalb der Seitenentnahmestelle (3) zusätzlich Prozessstabilisator (24), insbesondere PTZ, zugegeben.

Alle Druckangaben beziehen sich auf den Absolutdruck.

Alle ppm-Angaben beziehen sich auf das Gewicht (Gew.-ppm).

Ein 'Niedersieder' (bezogen auf Butylacrylat) ist ein Stoff, dessen Siedetemperatur niedriger als die Siedetemperatur des betreffenden Butylacrylats, also n-Butylacrylat bzw. iso-Butylacrylat, bei gleichem Druck ist.

Ein 'Höhersieder' (bezogen auf Butylacrylat) ist ein Stoff, dessen Siedetemperatur höher als die Siedetemperatur des betreffenden Butylacrylats, also n-Butylacrylat bzw. iso-Butylacrylat, bei gleichem Druck ist.

### Beispiele

Die Fahrweisen werden anhand der Daten aus einer thermodynamischen Simulation einer Gesamtanlage zur Herstellung von n-Butylacrylat dargestellt.

Die thermodynamische Simulation des Prozesses wurde mit der Software Aspen Plus® (kurz: Aspen) durchgeführt. Aspen ist eine umfangreiche Simulationssoftware, die zur Modellierung, Simulation und Optimierung chemischer Verfahren und Anlagen in der Industrie eingesetzt wird. Aspen verfügt über umfangreiche Modell-Datenbanken zur Modellierung der Basisoperationen sowie über Stoffdatenbanken für die Stoffeigenschaften vieler verschiedener Substanzen. Die Eigenschaften von Mischungen werden von Aspen mit Hilfe von unterschiedlichen thermodynamischen Modellen aus den Stoffdaten der reinen Substanzen berechnet.

### Beispiel 1

(Flüssigkeitsmengenverhältnis am oberen Ende der Trennwand (8), Verstärkungsteil (10) : Abtriebsteil (11) = 1 : 2 und
Mengenverhältnis der Brüdenströme am unteren Ende der Trennwand (8), Abtriebsteil (12) : Verstärkungsteil (13) = 1 : 1)

Ein Roh-n-Butylacrylat-Strom von 16000 kg/h mit einer Temperatur von 70 °C wurde flüssig auf der 28. theoretischen Trennstufe einer Trennwandkolonne (1) mit insgesamt 35 theoretischen Trennstufen eingespeist. Das Roh-n-Butylacrylat wies die folgende Zusammensetzung auf:

| | |
|---|---|
| n-Butylacrylat: | 86,4 Gew.-% |
| Wasser: | 1,1 Gew.-% |
| n-Butanol: | 3,7 Gew.-% |
| Di-n-butylether: | 0,33 Gew.-% |
| n-Butylacetat: | 2,2 Gew.-% |
| Butoxyester: | 5,0 Gew.-% |

weitere Höhersieder (bez. auf n-Butylacrylat): Rest

Die Trennwand (8) erstreckte sich vom 6. bis zur 30. theoretischen Trennstufe. Die Seitenentnahme (3) erfolgte auf der 15. theoretischen Trennstufe. Der Betrieb der Kolonne erfolgte bei einem Kopfdruck von 100 mbar und einem Sumpfdruck von 240 mbar.

Am Kopf der Kolonne wurde bei einer Temperatur von 35 °C kondensiert. Aus dem Kondensator (6) wurde ein dampfförmiger niedersiederhaltiger Strom (19) von 19 kg/h abgezogen. Aus dem kondensierten Strom wurde ein Teilstrom (4) von 1423 kg/h abgezogen. Die hochsiedenden Verunreinigungen (5) wurden am Kolonnensumpf in einem Mengenstrom von 1836 kg/h bei einer Temperatur von 117 °C entnommen. Am Seitenabzug wurde das Wertprodukt Rein-Butylacrylat bei einer Temperatur von 94 °C flüssig in einer Menge von 12722 kg/h erhalten.

Der Seitenabzug (3) wies die folgende Zusammensetzung auf:

| | |
|---|---|
| n-Butylacrylat: | 99,91 Gew.-% |
| Wasser: | < 0,01 Gew.-% |
| n-Butanol: | < 0,01 Gew.-% |
| Di-n-butylether: | 643 Gew.-ppm |
| n-Butylacetat: | 100 Gew.-ppm |

weitere Höhersieder (bez. auf n-Butylacrylat): Rest

Der Mindestgehalt an Acrylat von > 99,5 Gew.-% und die handelsüblichen Spezifikationen für die Nebenkomponenten n-Butylacetat mit 100 ppm und für Di-n-butylether mit 1000 ppm werden eingehalten. Die Destillationsausbeute für n-Butylacrylat lag bei über 92 %.

Das Flüssigkeitsmengenverhältnis für die Flüssigkeit am oberen Ende der Trennwand (8), Verstärkungsteil (10) : Abtriebsteil (11), betrug 1 : 2. Am unteren Ende der Trennwand (8) erfolgte die Aufteilung der Brüdenstrommengen, Abtriebsteil (12) : Verstärkungsteil (13), im Verhältnis 1 : 1. Die Heizleistung des Verdampfers betrug 3224 kW.

Durch das erfindungsgemäße Verfahren konnte die Destillation von Roh-Butylacrylat zu Rein-Butylacrylat z.B. bei einer Jahreskapazität von 100.000 t unter Einhaltung der geforderten Spezifikationen mit einer Investitionskosteneinsparung von 25 % und einer Energiekosteneinsparung von 31 % gegenüber einem herkömmlichen zweistufigen Destillationsverfahren durchgeführt werden.

### Vergleichsbeispiel 1

(Flüssigkeitsmengenverhältnis am oberen Ende der Trennwand (8), Verstärkungsteil (10) : Abtriebsteil (11) = 1 : 7)

Ein Roh-n-Butylacrylat-Strom von 16000 kg/h mit einer Temperatur von 70 °C wurde flüssig auf der 28. theoretischen Trennstufe einer Trennwandkolonne (1) mit insgesamt 35 theoretischen Trennstufen eingespeist. Das Roh-n-Butylacrylat wies die folgende Zusammensetzung auf:

| | |
|---|---|
| n-Butylacrylat: | 86,4 Gew.-% |
| Wasser: | 1,1 Gew.-% |
| n-Butanol: | 3,7 Gew.-% |
| Di-n-butylether: | 0,33 Gew.-% |
| n-Butylacetat: | 2,2 Gew.-% |
| Butoxyester: | 5,0 Gew.-% |

weitere Höhersieder (bez. auf n-Butylacrylat): Rest

Die Trennwand (8) erstreckte sich vom 6. bis zur 30. theoretischen Trennstufe. Die Seitenentnahme (3) erfolgte auf der 15. theoretischen Trennstufe. Der Betrieb der Kolonne erfolgte bei einem Kopfdruck von 100 mbar und einem Sumpfdruck von 240 mbar.

Am Kopf der Kolonne wurde bei einer Temperatur von 35 °C kondensiert. Aus dem Kondensator (6) wurde ein dampfförmiger niedersiederhaltiger Strom (19) von 19 kg/h abgezogen. Aus dem kondensierten Strom wurde ein Teilstrom (4) von 1411 kg/h abgezogen. Die hochsiedenden Verunreinigungen (5) wurden am Kolonnensumpf in einem Mengenstrom von 1850 kg/h bei einer Temperatur von 117 °C entnommen. Am Seitenabzug wurde das Wertprodukt Rein-Butylacrylat bei einer Temperatur von 94 °C flüssig in einer Menge von 12720 kg/h erhalten.

Der Seitenabzug (3) wies die folgende Zusammensetzung auf:

| | |
|---|---|
| n-Butylacrylat: | 99,83 Gew.-% |
| Wasser: | < 0,01 Gew.-% |
| n-Butanol: | < 0,01 Gew.-% |
| Di-n-butylether: | 1212 Gew.-ppm |
| n-Butylacetat: | 258 Gew.-ppm |
| Butoxyester: | < 0,01 Gew.-% |

weitere Höhersieder (bez. auf n-Butylacrylat): Rest

Der Mindestgehalt an Acrylat von > 99,5 Gew.-% wird eingehalten aber die handelsüblichen Spezifikationen für die Nebenkomponenten n-Butylacetat mit 100 ppm und für Di-n-butylether mit 1000 ppm werden nicht eingehalten.

Die Destillationsausbeute für n-Butylacrylat lag bei über 91 %.

Das Flüssigkeitsmengenverhältnis für die Flüssigkeit am oberen Ende der Trennwand (8), Verstärkungsteil (10) : Abtriebsteil (11), betrug 1 : 7. Am unteren Ende der Trennwand (8) erfolgte die Aufteilung der Brüdenstrommengen, Abtriebsteil (12) : Verstärkungsteil (13), im Verhältnis 1 : 1. Die Heizleistung des Verdampfers betrug 4524 kW.

### Vergleichsbeispiel 2

(Mengenverhältnis der Brüdenströme am unteren Ende der Trennwand (8), Abtriebsteil (12) : Verstärkungsteil (13) = 3 : 1)

Ein Roh-n-Butylacrylat-Strom von 16000 kg/h mit einer Temperatur von 70 °C wurde flüssig auf der 28. theoretischen Trennstufe einer Trennwandkolonne (1) mit insgesamt 35 theoretischen Trennstufen eingespeist. Das Roh-n-Butylacrylat wies die folgende Zusammensetzung auf:

| | |
|---|---|
| n-Butylacrylat: | 86,4 Gew.-% |
| Wasser: | 1,1 Gew.-% |
| n-Butanol: | 3,7 Gew.-% |
| Di-n-butylether: | 0,33 Gew.-% |
| n-Butylacetat: | 2,2 Gew.-% |
| Butoxyester: | 5,0 Gew.-% |

weitere Höhersieder (bez. auf n-Butylacrylat): Rest

Die Trennwand (8) erstreckte sich vom 6. bis zur 30. theoretischen Trennstufe. Die Seitenentnahme (3) erfolgte auf der 15. theoretischen Trennstufe. Der Betrieb der Kolonne erfolgte bei einem Kopfdruck von 100 mbar und einem Sumpfdruck von 240 mbar.

Am Kopf der Kolonne wurde bei einer Temperatur von 35 °C kondensiert. Aus dem Kondensator (6) wurde ein dampfförmiger niedersiederhaltiger Strom (19) von 18 kg/h abgezogen. Aus dem kondensierten Strom wurde ein Teilstrom (4) von 1236 kg/h abgezogen. Die hochsiedenden Verunreinigungen (5) wurden am Kolonnensumpf in einem Mengenstrom von 2026 kg/h bei einer Temperatur von 115 °C entnommen. Am Seitenabzug wurde das Wertprodukt Rein-Butylacrylat bei einer Temperatur von 93 °C flüssig in einer Menge von 12720 kg/h erhalten.

Der Seitenabzug (3) wies die folgende Zusammensetzung auf:

| | |
|---|---|
| n-Butylacrylat: | 98,75 Gew.-% |
| Wasser: | < 0,01 Gew.-% |
| n-Butanol: | < 0,4 Gew.-% |
| Di-n-butylether: | 1798 Gew.-ppm |
| n-Butylacetat: | 6640 Gew.-ppm |
| Butoxyester: | < 0,01 Gew.-ppm |

weitere Höhersieder (bez. auf n-Butylacrylat): Rest

Der Mindestgehalt an Acrylat von > 99,5 Gew.-% und die handelsüblichen Spezifikationen für die Nebenkomponenten n-Butylacetat mit 100 ppm und für Di-n-butylether mit 1000 ppm werden nicht eingehalten.

Die Destillationsausbeute für n-Butylacrylat lag bei über 90 %.

Das Flüssigkeitsmengenverhältnis für die Flüssigkeit am oberen Ende der Trennwand (8), Verstärkungsteil (10) : Abtriebsteil (11), betrug 1 : 2. Am unteren Ende der Trennwand (8) erfolgte die Aufteilung der Brüdenstrommengen, Abtriebsteil (12) : Verstärkungsteil (13), im Verhältnis 3 : 1. Die Heizleistung des Verdampfers betrug 3824 kW.

## Patentansprüche

1. Verfahren zur destillativen Gewinnung von Rein-Butylacrylat aus Roh-Butylacrylat, wobei Butyl für n-Butyl oder iso-Butyl steht, **dadurch gekennzeichnet, dass** das Verfahren in einer Trennwandkolonne (1) mit trennwirksamen Einbauten und Verdampfer (7) durchgeführt wird, in der eine Trennwand (8) in Kolonnenlängsrichtung unter Ausbildung eines oberen gemeinsamen Kolonnenbereichs (9), eines unteren gemeinsamen Kolonnenbereichs (14), eines Zulaufteils (10, 12) mit einer Seitenzulaufstelle (2) und eines Entnahmeteils (11, 13) mit einer Seitenentnahmestelle (3) angeordnet ist, die Kolonne eine Anzahl von theoretischen Trennstufen im Bereich von 20 bis 80 aufweist, wobei die Anzahl der theoretischen Trennstufen der Trennwandkolonne (1) sich auf die Summe der theoretischen Trennstufen im gemeinsamen oberen Kolonnenbereich (9), dem gemeinsamen unteren Kolonnenbereich (14) und dem Zulaufteil (10, 12) bezieht, die Seitenzulaufstelle (2) für das Roh-Butylacrylat auf einer theoretischen Trennstufe im Bereich beginnend mindestens zwei theoretische Trennstufen oberhalb der untersten und endend mindestens zwei theoretische Trennstufen unterhalb der obersten theoretischen Trennstufe angeordnet ist, die Seitenentnahmestelle (3) für das Rein-Butylacrylat auf einer theoretischen Trennstufe im Bereich beginnend mindestens zwei theoretische Trennstufen oberhalb der untersten und endend mindestens zwei theoretische Trennstufen unterhalb der obersten theoretischen Trennstufe angeordnet ist und die Trennwand (8) in der Kolonne im Bereich beginnend mindestens eine theoretische Trennstufe oberhalb der untersten und endend mindestens eine theoretische Trennstufe unterhalb der obersten theoretischen Trennstufe angeordnet ist, wobei man das Flüssigkeitsmengenverhältnis am oberen Ende der Trennwand (8) auf den Verstärkungsteil (10) und den Abtriebsteil (11) der Kolonne in einem Bereich von 1 : 0,2 bis 1 : 5 und das Mengenverhältnis der Brüdenströme am unteren Ende der Trennwand (8) auf den Abtriebsteil (12) und den Verstärkungsteil (13) der Kolonne in einem Bereich von 1 : 0,5 bis 1 : 2,0 einstellt, wobei es sich bei dem Butylacrylat um n-Butylacrylat handelt und das Roh-n-Butylacrylat die folgende Zusammensetzung aufweist:
40 bis 90 Gew.-% n-Butylacrylat,
0,1 bis 20 Gew.-% n-Butanol,
0,1 bis 20 Gew.-% Wasser,
0,1 bis 20 Gew.-% Höhersieder (bez. auf n-Butylacrylat),
0,1 bis 20 Gew.-% weitere Niedersieder (bez. auf n-Butylacrylat),
oder, wobei es sich bei dem Butylacrylat um iso-Butylacrylat handelt und das Roh-iso-Butylacrylat die folgende Zusammensetzung aufweist:
40 bis 90 Gew.-% iso-Butylacrylat,
0,1 bis 20 Gew.-% iso-Butanol,
0,1 bis 20 Gew.-% Wasser,
0,1 bis 20 Gew.-% Höhersieder (bez. auf iso-Butylacrylat),
0,1 bis 20 Gew.-% weitere Niedersieder (bez. auf iso-Butylacrylat).

2. Verfahren nach dem vorherigen Anspruch, **dadurch gekennzeichnet, dass** die Seitenzulaufstelle (2) für das Roh-Butylacrylat auf einer theoretischen Trennstufe im Bereich beginnend mindestens fünf theoretische Trennstufen oberhalb der untersten und endend mindestens fünf theoretische Trennstufen unterhalb der obersten theoretischen Trennstufe angeordnet ist, die Seitenentnahmestelle (3) für das Rein-Butylacrylat auf einer theoretischen Trennstufe im Bereich beginnend mindestens fünf theoretische Trennstufen oberhalb der untersten und endend mindestens fünf theoretische Trennstufen unterhalb der obersten theoretischen Trennstufe angeordnet ist und die Trennwand (8) in der Kolonne im Bereich beginnend mindestens vier theoretische Trennstufen oberhalb des untersten und endend mindestens vier theoretische Trennstufen unterhalb der obersten theoretischen Trennstufe angeordnet ist.

3. Verfahren nach Anspruch 1, **dadurch gekennzeichnet dass**, die Kolonne (1) eine Anzahl von theoretischen Trennstufen im Bereich von 30 bis 40 aufweist, die Seitenzulaufstelle (2) für das Roh-Butylacrylat auf einer theoretischen Trennstufe im Bereich beginnend mindestens 20 theoretischen Trennstufen oberhalb der untersten und endend mindestens zwei theoretische Trennstufen unterhalb der obersten theoretischen Trennstufe angeordnet ist, die Seitenentnahmestelle (3) für das Rein-Butylacrylat auf einer theoretischen Trennstufe im Bereich beginnend mindestens 10 theoretische Trennstufen oberhalb der untersten und endend mindestens 10 theoretische Trennstufen unterhalb der obersten theoretischen Trennstufe angeordnet ist und die Trennwand (8) in der Kolonne im Bereich beginnend mindestens fünf theoretische Trennstufen oberhalb des untersten und endend mindestens fünf theoretische Trennstufen unterhalb der obersten theoretischen Trennstufe angeordnet ist.

4. Verfahren nach einem der vorherigen Ansprüche, **dadurch gekennzeichnet, dass** die gegenüberliegende Seitenentnahmestelle (3) mindestens eine theoretische Trennstufe unterhalb der Seitenzulaufstelle (2) liegt, wobei im Fall verschiedener Anzahl an theoretischen Trennstufen im Entnahmeteil (11, 13) wie im Zulaufteil (10, 12) zur Zählung der Anzahl theoretischer Trennstufen für die Festlegung der relativen Höhenposition von Zulauf- und Entnahmestelle diejenige Seite mit der höheren Gesamtanzahl an Trennstufen im Bereich der Trennwand (8) herangezogen wird.

5. Verfahren nach einem der vorherigen Ansprüche, **dadurch gekennzeichnet, dass** als trennwirksame Einbauten Füllkörper, geordnete Packungen und/oder Böden vorgesehen sind.

6. Verfahren nach dem vorherigen Anspruch, **dadurch gekennzeichnet, dass** als Böden Dual-Flow-Böden eingesetzt werden.

7. Verfahren nach dem vorherigen Anspruch, **dadurch gekennzeichnet, dass** die Dual-Flow-Böden auf der Zulauf- und Entnahmeseite unterschiedliche Öffnungsverhältnisse zur Einstellung der optimalen Gasverteilung auf beiden Seiten der Trennwand (8) aufweisen.

8. Verfahren nach einem der vorherigen Ansprüche, **dadurch gekennzeichnet, dass** man die Verweilzeit im Verdampfer (7) und dem zugehörigen Rohrleitungssystem auf 1 bis 60 Minuten begrenzt.

9. Verfahren nach einem der vorherigen Ansprüche, **dadurch gekennzeichnet, dass** man das Flüssigkeitsmengenverhältnis am oberen Ende der Trennwand (8) auf den Verstärkungsteil (10) und den Abtriebsteil (11) der Kolonne in einem Bereich von 1 : 0,5 bis 1 : 2 einstellt.

10. Verfahren nach einem der vorherigen Ansprüche, **dadurch gekennzeichnet, dass** man das Mengenverhältnis der Brüdenströme am unteren Ende der Trennwand (8) auf den Abtriebsteil (12) und den Verstärkungsteil (13) der Kolonne in einem Bereich von 1 : 0,9 bis 1 : 1,5 einstellt.

11. Verfahren nach einem der vorherigen Ansprüche, **gekennzeichnet durch** einen Druck am Kolonnenkopf im Bereich von 20 mbar bis 5 bar.

12. Verfahren nach einem der vorherigen Ansprüche, **gekennzeichnet durch** eine Temperaturregelung im oberen gemeinsamen Kolonnenbereich (9), mittels eines Temperatursignals unterhalb der obersten theoretischen Trennstufe, die als Stellgröße den Destillatstrom, das Rücklaufverhältnis oder die Rücklaufmenge nutzt.

13. Verfahren nach einem der vorherigen Ansprüche, **gekennzeichnet durch** eine Temperaturregelung im unteren gemeinsamen Kolonnenbereich (14), mittels eines Temperatursignals oberhalb der untersten theoretischen Trennstufe, die als Stellgröße die Sumpfentnahmemenge nutzt.

14. Verfahren nach einem der vorherigen Ansprüche, **gekennzeichnet durch** eine Standregelung am Kolonnensumpf, die als Stellgröße die Seitenentnahmemenge nutzt.

15. Verfahren nach einem der vorherigen Ansprüche, gekennzeichnet, dass das Verhältnis der Querschnittsflächen des Bereichs des Entnahmeteils (11, 13) zum Bereich des Zulaufteils (10, 12) bei 4 : 1 bis 1 : 4 liegt.

16. Verfahren nach einem der vorherigen Ansprüche 1 bis 14, gekennzeichnet, dass das Verhältnis der Querschnittsflächen des Bereichs des Entnahmeteils (11, 13) zum Bereich des Zulaufteils (10, 12) bei 1,5 : 1 bis 1 : 1,5 liegt.

17. Verfahren nach einem der vorherigen Ansprüche zur destillativen Gewinnung von Rein-Butylacrylat mit einer Reinheit von ≥ 98,5 Gew.-%.

18. Verfahren nach einem der vorhergehenden Ansprüche, wobei das Roh-n-Butylacrylat die folgende Zusammensetzung aufweist:
60 bis 90 Gew.-% n-Butylacrylat,
1 bis 10 Gew.-% n-Butanol,
0,5 bis 10 Gew.-% Wasser,
1 bis 10 Gew.-% Höhersieder (bez. auf n-Butylacrylat),
1 bis 10 Gew.-% weitere Niedersieder (bez. auf n-Butylacrylat).

19. Verfahren nach einem der vorherigen Ansprüche 1 bis 17, wobei das Roh-iso-Butylacrylat die folgende Zusammensetzung aufweist:
60 bis 90 Gew.-% iso-Butylacrylat,
1 bis 10 Gew.-% iso-Butanol,
0,5 bis 10 Gew.-% Wasser,
1 bis 10 Gew.-% Höhersieder (bez. auf iso-Butylacrylat),
1 bis 10 Gew.-% weitere Niedersieder (bez. auf iso-Butylacrylat).

20. Verfahren nach einem der vorherigen Ansprüche, **dadurch gekennzeichnet, dass** ein Stabilisator 1 in den Verstärkungsteil (10) des Zulaufteils (10, 12) zugegeben wird.

21. Verfahren nach dem vorhergehenden Anspruch, **dadurch gekennzeichnet, dass** es sich bei dem Stabilisator 1 um Phenothiazin (PTZ) handelt.

22. Verfahren nach einem der vorherigen Ansprüche, **dadurch gekennzeichnet, dass** ein Stabilisator 2 in den Behälter (20), der das Kondensat (26) auffängt, und/oder in die Leitung eines Quenchkreislaufs (21), wobei es sich hierbei um einen flüssigen Rückführstrom eines Teils des Kondensats in den Kondensator (6) handelt, und/oder am Kopf des Kondensators (6) zugegeben wird.

23. Verfahren nach dem vorhergehenden Anspruch, **dadurch gekennzeichnet, dass** es sich bei dem Stabilisator 2 um p-Methoxyphenol (MeHQ) handelt.

## Claims

1. A process for isolating pure butyl acrylate from crude butyl acrylate, where butyl is n-butyl or isobutyl, by distillation, wherein the process is carried out in a divided wall column (1) which has separation-active internals and a vaporizer (7) and in which a dividing wall (8) is arranged in the longitudinal direction of the column to form an upper joint column region (9), a lower joint column region (14), an inflow section (10, 12) having a side feed point (2) and an offtake section (11, 13) having a side offtake point (3), the column has a number of theoretical plates in the range from 20 to 80, where the number of theoretical plates of the dividing wall column (1) relates to the sum of the theoretical plates in the joint upper column region (9), the joint lower column region (14) and the inflow section (10, 12), the side feed point (2) for the crude butyl acrylate is arranged at a theoretical plate in the region commencing at least two theoretical plates above the bottommost theoretical plate and ending at least two theoretical plates below the uppermost theoretical plate, the side offtake point (3) for the pure butyl acrylate is arranged at a theoretical plate in the region commencing at least two theoretical plates above the bottommost theoretical plate and ending at least two theoretical plates below the uppermost theoretical plate and the dividing wall (8) is arranged in the column in the region commencing at least one theoretical plate above the bottommost theoretical plate and ending at least one theoretical plate below the uppermost theoretical plate, where the ratio of amount of liquid at the upper end of the dividing wall (8) going to the enrichment section (10) and the stripping section (11) of the column is set in the range from 1:0.2 to 1:5 and the ratio of amounts of the vapor streams at the lower end of the dividing wall (8) going to the stripping section (12) and the enrichment section (13) of the column is set in the range from 1:0.5 to 1:2.0, where the butyl acrylate is n-butyl acrylate and the crude n-butyl acrylate has the following composition:
from 40 to 90% by weight of n-butyl acrylate,
from 0.1 to 20% by weight of n-butanol,
from 0.1 to 20% by weight of water,
from 0.1 to 20% by weight of relatively high boilers (relative to n-butyl acrylate),
from 0.1 to 20% by weight of further low boilers (relative to n-butyl acrylate),
or the butyl acrylate is isobutyl acrylate and the crude isobutyl acrylate has the following composition:
from 40 to 90% by weight of isobutyl acrylate,
from 0.1 to 20% by weight of isobutanol,
from 0.1 to 20% by weight of water,
from 0.1 to 20% by weight of relatively high boilers (relative to isobutyl acrylate),
from 0.1 to 20% by weight of further low boilers (relative to isobutyl acrylate).

2. The process according to the preceding claim, wherein the side feed point (2) for the crude butyl acrylate is arranged at a theoretical plate in the region commencing at least five theoretical plates above the bottommost theoretical plate and ending at least five theoretical plates below the uppermost theoretical plate, the side offtake point (3) for the pure butyl acrylate is arranged at a theoretical plate in the region commencing at least five theoretical plates above the bottommost theoretical plate and ending at least five theoretical plates below the uppermost theoretical plate and the dividing wall (8) in the column is arranged in the region commencing at least four theoretical plates above the bottommost theoretical plate and ending at least four theoretical plates below the uppermost theoretical plate.

3. The process according to claim 1, wherein the column (1) has a number of theoretical plates in the range from 30 to 40, the side feed point (2) for the crude butyl acrylate is arranged at a theoretical plate in the range commencing at least 20 theoretical plates above the bottommost theoretical plate and ending at least two theoretical plates below the uppermost theoretical plate, the side offtake point (3) for the butyl acrylate is arranged at a theoretical plate in the range commencing at least 10 theoretical plates above the bottommost theoretical plate and ending at least 10 theoretical plates below the uppermost theoretical plate and the dividing wall (8) in the column is arranged in the region commencing at least five theoretical plates above the bottommost theoretical plate and ending at least five theoretical plates below the uppermost theoretical plate.

4. The process according to any of the preceding claims, wherein the opposite side offtake point (3) is located at least one theoretical plate below the side feed point (2), where, in the case of different numbers of theoretical plates in the offtake section (11, 13) and the inflow section (10, 12), the side having the greatest total number of theoretical plates in the region of the dividing wall (8) is employed for counting the number of theoretical plates for determining the relative height position of feed point and offtake point.

5. The process according to any of the preceding claims, wherein random packing elements, ordered packing and/or trays are provided as separation-active internals.

6. The process according to the preceding claim, wherein dual-flow trays are used as trays.

7. The process according to the preceding claim, wherein the dual-flow trays on the inflow side and the offtake side have different opening ratios for setting the optimal gas distribution over the two sides of the dividing wall (8).

8. The process according to any of the preceding claims, wherein the residence time in the vaporizer (7) and the associated piping system is limited to from 1 to 60 minutes.

9. The process according to any of the preceding claims, wherein the ratio of amounts of liquid at the upper end of the dividing wall (8) going to the enrichment section (10) and the stripping section (11) of the column is set in the range from 1:0.5 to 1:2.

10. The process according to any of the preceding claims, wherein the ratio of amounts of the vapor streams at the lower end of the dividing wall (8) going to the stripping section (12) and the enrichment section (13) of the column is set in the range from 1:0.9 to 1:1.5.

11. The process according to any of the preceding claims, wherein the pressure at the top of the column is in the range from 20 mbar to 5 bar.

12. The process according to any of the preceding claims, wherein there is temperature regulation in the upper joint column region (9), by means of a temperature signal below the uppermost theoretical plate, which utilizes the distillate flow, the reflux ratio or the amount of reflux as manipulated variable.

13. The process according to any of the preceding claims, wherein there is temperature regulation in the lower joint column region (14), by means of a temperature signal above the bottommost theoretical plate, which utilizes the amount taken off at the bottom as manipulated variable.

14. The process according to any of the preceding claims, wherein there is level regulation at the bottom of the column which utilizes the amount taken off at the side as manipulated variable.

15. The process according to any of the preceding claims, wherein the ratio of the cross-sectional areas of the region of the offtake section (11, 13) to the region of the inflow section (10, 12) is from 4:1 to 1:4.

16. The process according to any of claims 1 to 14, wherein the ratio of the cross-sectional areas of the region of the offtake section (11, 13) to the region of the inflow section (10, 12) is from 1.5:1 to 1:1.5.

17. The process according to any of the preceding claims for isolating pure butyl acrylate having a purity of ≥98.5% by weight by distillation.

18. The process according to any of the preceding claims, wherein the crude n-butyl acrylate has the following composition:
from 60 to 90% by weight of n-butyl acrylate,
from 1 to 10% by weight of n-butanol,
from 0.5 to 10% by weight of water,
from 1 to 10% by weight of relatively high boilers (relative to n-butyl acrylate),
from 1 to 10% by weight of further low boilers (relative to n-butyl acrylate).

19. The process according to any of claims 1 to 17, wherein the crude isobutyl acrylate has the following composition:
from 60 to 90% by weight of isobutyl acrylate,
from 1 to 10% by weight of isobutanol,
from 0.5 to 10% by weight of water,
from 1 to 10% by weight of relatively high boilers (relative to isobutyl acrylate),
from 1 to 10% by weight of further low boilers (relative to isobutyl acrylate).

20. The process according to any of the preceding claims, wherein a stabilizer 1 is introduced into the enrichment section (10) of the inflow section (10, 12).

21. The process according to the preceding claim, wherein the stabilizer 1 is phenothiazine (PTZ).

22. The process according to any of the preceding claims, wherein a stabilizer 2 is introduced into the container (20) which collects the condensate (26) and/or into the conduit of a quenching circuit (21), where this is a liquid return stream of part of the condensate into the condenser (6), and/or at the top of the condenser (6) .

23. The process according to the preceding claim, wherein the stabilizer 2 is p-methoxyphenol (MeHQ).

## Revendications

1. Procédé pour l'obtention, par distillation, d'acrylate de butyle pur à partir d'acrylate de butyle pur, butyle représentant n-butyle ou iso-butyle, **caractérisé en ce que** le procédé est mis en œuvre dans une colonne à paroi de séparation (1) doté d'éléments encastrés efficaces pour la séparation et d'un évaporateur (7), dans lequel une paroi de séparation (8) est disposée dans la direction longitudinale de colonne avec formation d'un domaine de colonne (9) commun supérieur, d'un domaine de colonne (14) commun inférieur, d'un élément d'alimentation (10, 12) doté d'une entrée latérale (2) et d'un élément de prélèvement (11, 13) doté d'un point de prélèvement latéral (3), la colonne présentant un nombre de plateaux théoriques de séparation dans la plage de 20 à 80, le nombre de plateaux théoriques de séparation de la colonne à paroi de séparation (1) se rapportant à la somme des plateaux théoriques de séparation dans le domaine de colonne (9) supérieur commun, du domaine de colonne (14) inférieur commun et de l'élément d'alimentation (10, 12), l'entrée latérale (2) pour l'acrylate de butyle brut étant disposée sur un plateau théorique de séparation dans le domaine commençant au moins deux plateaux théoriques de séparation au-dessus du plateau théorique de séparation le plus en dessous et finissant au moins deux plateaux théoriques de séparation au-dessous du plateau théorique de séparation le plus au-dessus, le point de prélèvement latéral (3) pour l'acrylate de butyle pur étant disposé sur un plateau théorique de séparation dans le domaine commençant au moins deux plateaux théoriques de séparation au-dessus du plateau théorique de séparation le plus en dessous et finissant au moins deux plateaux théoriques de séparation au-dessous du plateau théorique de séparation le plus au-dessus et la paroi de séparation (8) étant disposée dans la colonne dans le domaine commençant au moins un plateau théorique de séparation au-dessus du plateau théorique de séparation le plus en dessous et finissant au moins un plateau théorique de séparation au-dessous du plateau théorique de séparation le plus au-dessus, dans lequel on ajuste le rapport de quantités de liquide au niveau de l'extrémité supérieure de la paroi de séparation (8) sur l'élément de renforcement (10) et la partie de rectification (11) de la colonne dans une plage de 1:0,2 à 1:5 et le rapport de quantité des flux de vapeurs au niveau de l'extrémité inférieure de la paroi de séparation (8) sur la partie de rectification (12) et l'élément de renforcement (13) de la colonne dans une plage de 1:0,5 à 1:2,0, l'acrylate de butyle étant l'acrylate de n-butyle et l'acrylate de n-butyle brut présentant la composition suivante :
40 à 90 % en poids d'acrylate de n-butyle,
0,1 à 20 % en poids de n-butanol,
0,1 à 20 % en poids d'eau,
0,1 à 20 % en poids de composés volatiles (par rapport à l'acrylate de n-butyle),
0,1 à 20 % en poids de composés non volatiles supplémentaires (par rapport à l'acrylate de n-butyle),
ou, l'acrylate de butyle étant l'acrylate d'iso-butyle et l'acrylate d'iso-butyle brut présentant la composition suivante :
40 à 90 % en poids d'acrylate d'iso-butyle,
0,1 à 20 % en poids d'iso-butanol,
0,1 à 20 % en poids d'eau,
0,1 à 20 % en poids de composés volatiles (par rapport à l'acrylate d'iso-butyle),
0,1 à 20 % en poids de composés non volatiles supplémentaires (par rapport à l'acrylate d'iso-butyle).

2. Procédé selon la revendication précédente, **caractérisé en ce que** l'entrée latérale (2) pour l'acrylate de butyle brut est disposée sur un plateau théorique de séparation dans le domaine commençant au moins cinq plateaux théoriques de séparation au-dessus du plateau théorique de séparation le plus en dessous et finissant au moins cinq plateaux théoriques de séparation au-dessous du plateau théorique de séparation le plus au-dessus, le point de prélèvement latéral (3) pour l'acrylate de butyle pur est disposé sur un plateau théorique de séparation dans le domaine commençant au moins cinq plateaux théoriques de séparation au-dessus du plateau théorique de séparation le plus en dessous et finissant au moins cinq plateaux théoriques de séparation au-dessous du plateau théorique de séparation le plus au-dessus et la paroi de séparation (8) dans la colonne est disposée dans le domaine commençant au moins quatre plateaux théoriques de séparation au-dessus du plateau théorique de séparation le plus en dessous et finissant au moins quatre plateaux théoriques de séparation au-dessous du plateau théorique de séparation le plus au-dessus.

3. Procédé selon la revendication 1, **caractérisé en ce que** la colonne (1) présente un nombre de plateaux théoriques de séparation dans la plage de 30 à 40, l'entrée latérale (2) pour l'acrylate de butyle brut est disposée sur un plateau théorique de séparation dans le domaine commençant au moins 20 plateaux théoriques de séparation au-dessus du plateau théorique de séparation le plus en dessous et finissant au moins deux plateaux théoriques de séparation au-dessous du plateau théorique de séparation le plus au-dessus, le point de prélèvement latéral (3) pour l'acrylate de butyle pur est disposé sur un plateau théorique de séparation dans le domaine commençant au moins 10 plateaux théoriques de séparation au-dessus du plateau théorique de séparation le plus en dessous et finissant au moins 10 plateaux théoriques de séparation au-dessous du plateau théorique de séparation le plus au-dessus et la paroi de séparation (8) dans la colonne est disposée dans le domaine commençant au moins cinq plateaux théoriques de séparation au-dessus du plateau théorique de séparation le plus en dessous et finissant au moins cinq plateaux théoriques de séparation au-dessous du plateau théorique de séparation le plus au-dessus.

4. Procédé selon l'une quelconque des revendications précédentes, **caractérisé en ce que** le point de prélèvement latéral (3) opposé se situe au moins un plateau théorique de séparation au-dessous de l'entrée latérale (2), dans lequel dans le cas d'un nombre différent de plateaux théoriques de séparation dans l'élément de prélèvement (11, 13) par rapport à l'élément d'alimentation (10, 12), pour le décompte du nombre de plateaux théoriques de séparation pour l'établissement de la position relative en hauteur du point d'alimentation et du point de prélèvement, le côté doté du nombre total de plateaux de séparation le plus élevé dans le domaine de la paroi de séparation (8) est considéré.

5. Procédé selon l'une quelconque des revendications précédentes, **caractérisé en ce qu'**en tant qu'éléments encastrés efficaces, des corps de charge, des emballages ordonnés et/ou des sols sont prévus.

6. Procédé selon la revendication précédente, **caractérisé en ce qu'**en tant que sols, des sols à double écoulement sont utilisés.

7. Procédé selon la revendication précédente, **caractérisé en ce que** les sols à double écoulement sur le côté d'alimentation et le côté de prélèvement présentent différents rapports d'ouverture pour l'ajustement de la répartition de gaz optimale sur les deux côtés de la paroi de séparation (8).

8. Procédé selon l'une quelconque des revendications précédentes, **caractérisé en ce qu'**on limite le temps de séjour dans l'évaporateur (7) et dans le système de conduites tubulaires associé à 1 à 60 minutes.

9. Procédé selon l'une quelconque des revendications précédentes, **caractérisé en ce qu'**on ajuste le rapport de quantités de liquide au niveau de l'extrémité supérieure de la paroi de séparation (8) sur l'élément de renforcement (10) et la partie de rectification (11) de la colonne dans une plage de 1:0,5 à 1:2.

10. Procédé selon l'une quelconque des revendications précédentes, **caractérisé en ce qu'**on ajuste le rapport de quantité des flux de vapeurs au niveau de l'extrémité inférieure de la paroi de séparation (8) sur la partie de rectification (12) et l'élément de renforcement (13) de la colonne dans une plage de 1:0,9 à 1:1,5.

11. Procédé selon l'une quelconque des revendications précédentes, **caractérisé par** une pression au niveau de la tête de colonne dans la plage de 20 mbars à 5 bars.

12. Procédé selon l'une quelconque des revendications précédentes, **caractérisé par** une régulation de température au niveau du domaine de colonne (9) commun supérieur, au moyen d'un signal de température au-dessous du plateau théorique de séparation le plus au-dessus, qui utilise en tant que grandeur de réglage le fluide de distillat, le rapport de retour ou la quantité de retour.

13. Procédé selon l'une quelconque des revendications précédentes, **caractérisé par** une régulation de température dans le domaine de colonne (14) commun inférieur, au moyen d'un signal de température au-dessus du plateau théorique de séparation le plus en dessous, qui utilise en tant que grandeur de réglage la quantité de prélèvement de fond.

14. Procédé selon l'une quelconque des revendications précédentes, **caractérisé par** une position réglée au niveau du fond de colonne qui utilise en tant que grandeur de réglage la quantité de prélèvement latéral.

15. Procédé selon l'une quelconque des revendications précédentes, **caractérisé en ce que** le rapport des surfaces de section transversale du domaine de l'élément de prélèvement (11, 13) sur le domaine de l'élément d'alimentation (10, 12) se situe de 4:1 à 1:4.

16. Procédé selon l'une quelconque des revendications précédentes 1 à 14, **caractérisé en ce que** le rapport des surfaces de section transversale du domaine de l'élément de prélèvement (11, 13) sur le domaine de l'élément d'alimentation (10, 12) se situe de 1,5:1 à 1:1,5.

17. Procédé selon l'une quelconque des revendications précédentes pour l'obtention par distillation d'acrylate de butyle pur doté d'une pureté ≥ 98,5 % en poids.

18. Procédé selon l'une quelconque des revendications précédentes, l'acrylate de n-butyle brut présentant la composition suivante :
60 à 90 % en poids d'acrylate de n-butyle,
1 à 10 % en poids de n-butanol,
0,5 à 10 % en poids d'eau,
1 à 10 % en poids de composés volatiles (par rapport à l'acrylate de n-butyle),
1 à 10 % en poids de composés non volatiles supplémentaires (par rapport à l'acrylate de n-butyle).

19. Procédé selon l'une quelconque des revendications précédentes 1 à 17, l'acrylate d'iso-butyle brut présentant la composition suivante : 60 à 90 % en poids d'acrylate d'iso-butyle,
1 à 10 % en poids d'iso-butanol,
0,5 à 10 % en poids d'eau,
1 à 10 % en poids de composés volatiles (par rapport à l'acrylate d'iso-butyle),
1 à 10 % en poids de composés non volatiles supplémentaires (par rapport à l'acrylate d'iso-butyle).

20. Procédé selon l'une quelconque des revendications précédentes, **caractérisé en ce qu'**un stabilisant 1 est ajouté dans l'élément de renforcement (10) de l'élément d'alimentation (10, 12).

21. Procédé selon la revendication précédente, **caractérisé en ce que** le stabilisant 1 est une phénothiazine (PTZ).

22. Procédé selon l'une quelconque des revendications précédentes, **caractérisé en ce qu'**un stabilisant 2 est ajouté dans le récipient (20), qui réceptionne le condensat (26), et/ou dans la conduite d'un circuit de trempage (21), qui est un fluide de retour liquide d'une partie du condensat dans le condensateur (6), et/ou au niveau de la tête du condensateur (6).

23. Procédé selon la revendication précédente, **caractérisé en ce que** le stabilisant 2 est le p-méthoxyphénol (MeHQ).
